# EUROPEAN PATENT SPECIFICATION

(11) **EP 0 140 434 B1**
(45) Date of publication and mention of the grant of the patent: **04.08.1993**
(21) Application number: 84201447.4
(22) Date of filing: 09.10.1984
(51) Int. Cl.: A61K 9/06, A61K 9/72

(54) **Pharmaceutical composition with systemic anticholineesterasic, agonistic-cholinergic and antimuscarinic activity**
Arzneimittel mit systematischer anticholinesterasischer, agonistisch-cholinergischer und antimuskariner Wirkung
Composition pharmaceutique à activité anticholinestérasique, agonistique-cholinergique et antimuscarinique

(30) Priority: 21.10.1983 IT 2339683
(43) Date of publication of application: 08.05.1985
(73) Proprietor: PRODOTTI FORMENTI S.r.l., 20149 Milano (IT)
(72) Inventor: Casadio, Silvano, I-20136 Milano (IT); Casadio, Vittorio, I-20122 Milano (IT)
(74) Representative: Appoloni, Romano

(56) References cited:
- EP-A- 0 131 315

## Description

Among the drugs of the autonomic nervous system, the parasympathomimetic drugs, and above all the anticholinesterasic and the antimuscarinic drugs, are important in the therapy of the illnesses of the gastroenteric apparatus characterized by spasm, gastric hypersecretion, hypermotility and in the therapy of atonies of the smooth muscle tissue of gastroenteric tract, of urinary vesica, and in the treatment of myasthenia gravis. Many of these parasympathomimetic drugs have the structure of quaternary ammonium salts (which will be denominated hereunder also as onium salts or compounds). Unfortunately, the bioavailability of onium compounds, administered by the oral way, is nearly always unsatisfactory, and however much lower than that consequent to the administration by the parenteral way.

This insufficient bioavailability of the onium salts under examination is evidenced by the large differences in LD₅₀ observed as a function of the various administration ways (from literature references).

For the butylbromide of scopolamine the following LD₅₀ values are e.g. reported for mice:
15,6 mg/kg by intravenous way
74 mg/kg by parenteral way
570 mg/kg by subcutaneous way
3.000 mg/kg by oral way.

Such values are, always for mice, respectively as follows, for prifinium bromide:
11 mg/kg by intravenous way
43 mg/kg by parenteral way
30 mg/kg by subcutaneous way.
330 mg/kg by oral way.

The large differences in absorption and bioavailability observed as a function of the different administration ways means, in the therapeutical practice, that oral dosages should be much higher than parenteral dosages. Neostigmine, e.g., is prescribed for parenteral administering in 0,5 mg-vials (on the average, 3 vials per day), whilst the lozenge dosage is 15 mg per lozenge, and the daily dosage may be as high as 20 lozenges.

Prifinium bromide dosage is 4 mg per vial, 25 mg per capsule, and 50 mg per suppository.

Tiemonium methylsulphate dosage is 4 mg per vial, 25 mg per lozenge, and 50 mg per suppository.

Emepronium bromide dosage is 50 mg per vial and 100 mg per lozenge.

The limited oral bioavailability of said onium salts is confirmed by the pharmacodynamic researches carried out on man, which have shown bioavailability rates by oral administration of the order of from 3 to 5% of those achieved by means of intravenous administration.

Among the causes which have been considered to be responsible for such large differences in bioavailability, the reduced penetration of onium salts through the gastrointestinal mucosa, with consequent inhibition to reach the smooth muscle cellular receptors, is one which has found experimental confirmation.

Onium salts are virtually insoluble in the lipoidal components of the membranes of the mucous cells of the gastrointestinal segment, and this is probably the most important barrier against the absorption of the same salts.

It must however be outlined that it is generally recognized that the absorption of ionized drugs by the intestine takes place in a reduced amount and in an unreliable way; this is particularly true for the drugs of the class of quaternary ammonium salts.

Finally, it is well known that the absorption through the gastrointestinal tract can be conditioned by the quantity and the nature of the food which is present inside the stomach, by the gastrointestinal motility, by the transit time, by the capability by the microbial flora to deactivate the active principle, and by the metabolizing connected with the first-pass effect.

Purpose of the present invention is to prepare novel pharmaceutical compositions of such drugs, which give therapeutical performances similar to those to be reached by the parenteral administration way, but which are cheaper and better acceptable by the patient.

In particular, according to the invention, the novel pharmaceutical compositions being searched for must produce a high bioavailability of the active principle and uniformity of hematic levels, properties which are absent in the compositions for oral use of the same active principles.

In order to achieve such purposes, the invention provides the use of neostigmine methylsulfate or tiemonium methylsulfate for the manufacture of a medicament in the form of an aqueous solution for exclusively nasal administration of the medicament in a minimum concentration of 30 mg/ml to obtain a basically agonistic-cholinergic or anticholinergic activity whilst reducing the bradycardiac side effect.

The structural formulae of the active principles in question are described hereunder,

### Neostigmine methylsulphate

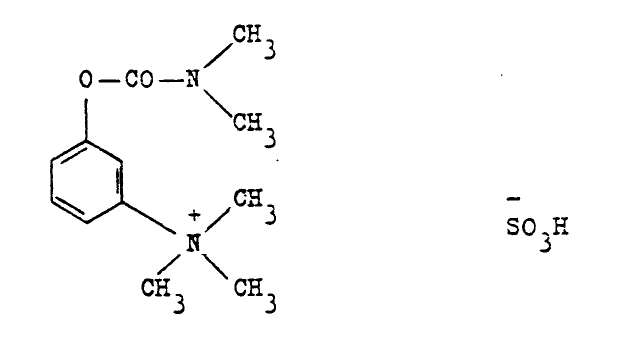

### Tiemonium methylsulphate

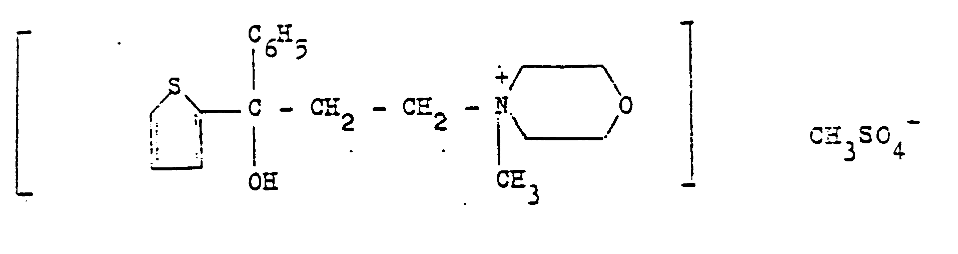

According to the invention, the active compounds above defined are prepared as pharmaceutical compositions with a nasal carrier, which renders them suitable to be administered by intranasal way, with considerably better results than obtained by using the compositions intended for oral and rectal use, as far as an increased bioavailability of the active principle, and the minimisation of the variations of the hematic levels of it are regarded, thus allowing these onium salts to be used at far lower dosage levels than usually employed for the use by oral and rectal way.

A surprising feature of the invention is that, very clearly, these onium salts are very rapidly absorbed from the nasal mucosa into the systemic hematic circulation, without first-pass metabolism. That is to say, an efficacious systemic anticholineesterasic and antimuscarinic therapeutical response is obtained.

Each one of the aforementioned onium salts can be conveniently administered by intranasal way to warm-blooded animals by means of formulations suitable for intranasal application, such formulations comprising the selected onium salt, in a suitable quantity to carry out the anticholineesterasic, agonist-cholinergic and antimuscarinic effect, together with a pharmaceutically acceptable and non-toxic nasal carrier.

The choice of the pharmaceutically acceptable and non-toxic carriers, which does not exclude the use of carriers traditionally mentioned in the art of nasal administering, depends on the chemical-physical characteristics of the onium salt, on the required dosages, on the selected type of formulation (solution, nasal gel, nasal ointment, aerosol spray, and so on), on the stability of the onium salt, etc.

The preferred dosage forms by the intranasal way are almost always solutions, dispersions in water base, such base being either gelled or not; in any case, water may represent the main ingredient of the formulations.

Minor quantities are employed in the formulations, of other ingredients, such as buffering agents, wetting agents, dispersing agents, pH adjustment agents, gelling agents and viscosity increasers.

Compatibly with the nature and the complexity of the formulation, is it preferred that the formulation be isotonic.

It has been found additionally that the carriers with prevailingly aqueous base and at low viscosity, as well as those carriers which use propellants based on halogenated hydrocarbons, tend to increase the absorption rate.

As examples of halogenated hydrocarbons, trichlorofluoromethane, dichlorofluoromethane, trichlorotrifluoroethane and dichlorotetrafluoroethane are preferred.

The carriers based on gelled supports, on O/W and W/O emulsions, used for intranasal application or through nebulizing, allow effects to be obtained, which are more durable in time, without significantly penalizing the rapidity of such effects.

Should the onium salts be not stable enough in the ready-for-application formulations, the active principle can be freeze-dried on a suitable support (mannitol, glycine, etc), which will be dissolved and/or dispersed by the suitable vehicle at the moment of the intranasal administering only.

Examples of the preparation of typical nasal compositions containing the above salts are reported herein-under. These Examples are reported for illustrative purposes only, and are not to be intended as limitative of the invention.

### EXAMPLES

### Example 1

### Aqueous solution of Neostigmine methylsulphate for intranasal nebulizing (pH 6,4)

| | % weight/volume |
|---|---|
| Neostigmine methylsulphate | 3 g |
| sodium chloride | 0,9 g |
| Monopotassic phosphate | 0,68 g |
| Sodium hydroxide | 0,056 g |
| Methyl p-hydroxybenzoate | 0,080 g |
| Propyl p-hydroxybenzoate | 0,020 g |
| Glycerin | 10 g |
| Depurated water, as much as necessary to | 100 ml |

The following products are dissolved in water, in the following order: neostigmine metylsulphate, sodium chloride, monopotassic phosphate, sodium hydroxide.

Methyl and propyl p-hydroxybenzoates are dissolved in glycerin, this solution is then added to the preceding one, carefully stirring.

The solution is administered by using nebulizers with pneumatic pump, and distributing valve rated at 50 - 100 microlitres per each nebulizing.

### Example 2 (comparative)

### Aqueous solution of tiemonium iodide for intranasal nebulizing (pH 6,4)

| | % weight/volume |
|---|---|
| Tiemonium iodide | 4 g |
| Sodium chloride | 0,9 g |
| monopotassic phosphate | 0,68 g |
| sodium hydroxide | 0,056 g |
| methyl p-hydroxybenzoate | 0,080 g |
| propyl p-hydroxybenzoate | 0,020 g |
| glycerin | 10 g |
| Propylene glycol | 20 g |
| Depurated water, as much as necessary to | 100 ml |

The following products are dissolved in water, in the following order: tiemonium iodide; then sodium chloride, monopotassic phosphate, sodium hydroxide.

Methyl p-hydroxybenzoate and propyl p-hydroxy-benzoate are dissolved in glycerin and propylene glycol; when the solution is complete, it is added to the preceding one.

As for the therapeutical application, see Example 1.

### Example 3

### Aqueous solution of neostigmine methylsulphate, with increased viscosity, for intranasal nebulizing (pH 6,5)

| | % weight/volume |
|---|---|
| Neostigmine methylsulphate | 3 g |
| sodium chloride | 0,9 g |
| monobasic phosphate | 0,680 g |
| sodium hydroxide | 0,056 g |
| methyl p-hydroxybenzoate | 0,080 g |
| propyl p-hydroxybenzoate | 0,020 g |
| Hydroxypropylmethylcellulose | 0,500 g |
| Depurated water, as much as necessary to | 100 ml |

Methyl p-hydroxybenzoate and propyl p-hydroxybenzoate are dissolved in heated water; after cooling, neostigmine methylsulphate, sodium chloride, monopotassic phosphate, sodium hydroxide and hydroxypropylmethylcellulose are dissolved, in the order as shown.

Also this solution is preferably applied by means of nebulizers.

### Example 4 (comparative)

### Aqueous solution of tiemonium iodide, with increased viscosity, for intranasal nebulizing (pH 6,5)

| | % weight/volume |
|---|---|
| Tiemonium iodide | 4 g |
| sodium chloride | 0,9 g |
| monobasic phosphate | 0,680 g |
| sodium hydroxide | 0,056 g |
| methyl p-hydroxybenzoate | 0,080 g |
| propyl p-hydroxybenzoate | 0,020 g |
| hydroxypropylmethylcellulose | 0,500 g |
| Glycerin | 10 g |
| Propylene glycol | 20 g |
| Depurated water, as much as necessary to | 100 ml |

Tiemonium iodide , then sodium chloride, monopotassic phosphate, sodium hydroxide, hydroxypropylmethylcellulose are dissolved in water, in the order shown.

Methyl p-hydroxybenzoate and propyl p-hydroxybenzoate are dissolved in propylene glycol and glycerin; upon completion, this solution is added to the preceding one.

This formulation is applied as the solutions described in previous Examples 1, 2 and 3.

### Example 5

### Nasal gel of neostigmine methylsulphate (pH 7)

| | % weight/volume |
|---|---|
| Neostigmine methylsulphate | 2,5 g |
| Carboxypolymethylene | 1 g |
| propylene glycol | 20 g |
| methyl p-hydroxybenzoate | 0,08 g |
| propyl p-hydroxybenzoate | 0,02 g |
| triethanolamine | 1,1 g |
| depurated water, as much as necessary to | 100 ml |

In a share of water, neostigmine methylsulphate is added, and carboxypolymethylene is added.

Methyl p-hydroxybenzoate and propyl p-hydroxybenzoate are dissolved in propylene glycol; the thus obtained solution is added to the preceding one.

With the balance of water triethanolamine is dissolved; add this solution to the proceding one, mixing and stirring carefully.

The gel is applied as a normal ointment for nasal use.

### Example 6 (comparative)

### Nasal gel of tiemonium iodide (pH 7)

| | % weight/volume |
|---|---|
| Tiemonium iodide | 3,5 g |
| Carboxypolymethylene | 1,0 g |
| propylene glycol | 20 g |
| methyl p-hydroxybenzoate | 0,080 g |
| propyl p-hydroxybenzoate | 0,020 g |
| triethanolamine | 1,1 g |
| depurated water, as much as necessary to | 100 ml |

In a share of water tiemonium iodide is dissolved, and carboxypolymethylene is then added.

Propylene glycol, methyl p-hydroxybenzoate, propyl p-hydroxybenzoate are completely dissolved; the so obtained solution is added to the preceding one.

With the balance of water, a solution is prepared of triethanolamine, such solution is carefully mixed and is then added to the other solution.

The gel is applied as a normal ointment for nasal use.

### Example 7

### Oily suspension of neostigmine methylsulphate for intranasal application (pH 6,3)

| | % weight/volume |
|---|---|
| Neostigmine methylsulphate | 3 g |
| Triglycerids of vegetable fatty acids, as much as necessary to | 100 ml |

The particles of neostigmine methylsulphate shall have an average diameter of 10µ . pH 6,3.

The dispersion is carried out by any traditional method, such as colloid mill, and so on.

The dispersion is applied by nasal instillation, as the following Example too.

### Example 8 (comparative)

### Oily suspension of tiemonium iodide (pH 6,3)

| | % weight/volume |
|---|---|
| Tiemonium iodide | 4 g |
| Triglycerids of vegetable fatty acids, as much as necessary to | 100 ml |

Particle diameter 10µ .

The suspension is prepared by dispersing the active principle in the oil, by any traditional system, such as colloid mill for example.

### Example 9

### Freeze-dried composition of neostigmine methylsulphate (pH 5,6)

| | % weight/volume |
|---|---|
| Neostigmine methylsulphate | 3 g |
| sodium chloride | 0,9 g |
| mannitol | 10 g |
| depurated water, as much as necessary to | 100 ml |

Mannitol, sodium chloride, neostigmine are dissolved in water.

The solution is distributed in vials, is chilled and is submitted to the freeze-drying procedure.

At the moment of use, the solution is restored, for intranasal nebulizing, by means of depurated water.

### Example 10 (comparative)

### Freeze-dried composition of tiemonium iodide (pH 5,6)

| | % weight/volume |
|---|---|
| Tiemonium iodide | 4 g |
| sodium chloride | 0,9 g |
| mannitol | 10,0 g |
| depurated water, as much as necessary to | 100 ml |

Mannitol, sodium chloride and tiemonium iodide are dissolved in water.

The solution is distributed in vials, is chilled, and is submitted to the freeze-drying procedure.

At the moment of use, the solution is restored, by means of depurated water, which allow the intranasal nebulizing to be carried out.

### Example 11

### Salve for nasal application of neostigmine methyl-sulphate (pH 6,2)

| | % weight/volume |
|---|---|
| Neostigmine methylsulphate | 3 g |
| carboxypolymethylene | 1 g |
| triethanolamine | 1,25 g |
| methyl p-hydroxybenzoate | 0,08 g |
| propyl p-hydroxybenzoate | 0,02 g |
| liquid paraffin | 10,00 g |
| vaseline oil | 10,00 g |
| castor oil P.O.E. | 5,00 g |
| Depurated water as much as necessary to | 100 g |

In a share of the water, neostigmine methylsulphate is dissolved and then, under stirring, carboxypolymethylene.

The solution of triethanolamine in the balance of water is then added: the solution is thoroughly mixed and heated at 65°C.

A separated solution has been prepared by heating at 65°C liquid paraffin, vaseline oil and castor oil.

The oily solution is added to the aqueous one, and, the stirring is continued, while slowly cooling down to room temperature.

The composition is applied as a normal ointment for nasal use.

### Example 12 (comparative)

### Nasal salve of tiemonium iodide (pH 6,2)

| | % weight/volume |
|---|---|
| Tiemonium iodide | 4 g |
| carboxypolymethylene | 1 g |
| triethanolamine | 1,25 g |
| methyl p-hydroxybenzoate | 0,080 g |
| propyl p-hydroxybenzoate | 0,020 g |
| liquid paraffin | 10,00 g |
| vaseline oil | 10,00 g |
| castor oil P.O.E. | 5,00 g |
| depurated water, as much as necessary to | 100 g |

In a share of the water the tiemonium iodide is dissolved, and, with stirring, the carboxypolymethylene; the solution is stirred until completion.

The solution of water/triethanolamine is then added. The total solution is heated at 65°C.

Liquid paraffin, vaseline oil and castor oil P.O.E. are heated at 65°C.

While being stirred, the aqueous and the oily solutions are combined.

Stirring is continued until the temperature has decreased down to room temperature.

This formulation is applied as a normal ointment for nasal use.

### Example 13

### Pressurized aerosol of neostigmine methylsulphate

| | % weight/volume |
|---|---|
| Neostigmine methylsulphate | 3 g |
| soy lecithin | 0,6 g |
| anhydrous ethanol | 5 g |
| Frigen 113 | 20 g |
| Frigen 11/12/114 | 71,4 g |

The neostigmine methylsulphate, previously pulverized to about 10 µ, is dispersed in anhydrous ethanol, soy lecithin and propellants are added, and the mixture is conditioned within aerosol bombs.

### Example 14

### Pressurized aerosol of tiemonium iodide (comparative)

| | % weight/volume |
|---|---|
| Tiemonium iodide | 4 g |
| soy lecithin | 0,6 g |
| anhydrous ethanol | 5 g |
| Frigen 113 | 20 g |
| Frigen 11/12/114 | 70,4 g |

Tiemonium iodide is dispersed in ethanol, then soy lecithin, Frigen 113 and Frigen 11/12/114 are added, and the mixture is conditioned inside aerosol bombs.

## Claims

1. Use of neostigmine methylsulphate or tiemonium methylsulphate for the manufacture of a medicament in the form of an aqueous solution for exclusively nasal administration of the medicament in a minimum concentration of 30 mg/ml to obtain a basically agonistic-cholinergic or anticholinergic activity whilst reducing the bradycardiac side effect.

2. Use according to claim 1, characterised by the fact that neostigmine methylsulphate is employed in order to cause an agonistic-cholinergic systemic effect.

3. Use according to claim 2, characterised by the fact that said medicament is suitable for the treatment of myasthenia by reducing bradycardiac side effects.

4. Use according to claim 1, characterised by the fact that tiemonium methylsulphate is employed in order to cause an anticholinergic systemic effect.

## Patentansprüche

1. Verwendung von Neostigminmethylsulfat oder Thiemoniummethylsulfat zur Herstellung eines Medikaments in Form einer wässrigen Lösung für ausschliesslich nasale Verabreichung des Medikaments in einer Minimalkonzentration von 30 mg/ml zum Erhalt einer im wesentlichen agonistisch-cholinergischen oder anticholinergischen Wirkung, während die bradykardialen Nebenwirkungen reduziert werden.

2. Verwendung gemäss Anspruch 1, dadurch **gekennzeichnet,** dass Neostigminmethylsulfat zur Bewirkung eines systemischen agonistisch-cholinergischen Effekts eingesetzt wird.

3. Verwendung gemäss Anspruch 2, dadurch **gekennzeichnet,** dass das Medikament geeignet ist zur Behandlung von Myasthenie durch Verringerung der bradykardialen Nebenwirkungen.

4. Verwendung gemäss Anspruch 1, dadurch **gekennzeichnet,** dass Thiemoniummethylsulfat eingesetzt wird, um einen systemischen anticholinergischen Effekt zu erzielen.

## Revendications

1. Utilisation du méthylsulfate de néostigmine ou du méthylsulfate de thiémonium pour la préparation d'un médicament sous la forme d'une solution aqueuse destinée exclusivement à une administration par voie nasale du médicament, à une concentration minimale de 30 mg/ml, pour l'obtention d'une activité fondamentalement agonistique-cholinergique ou anti-cholinergique, l'effet secondaire bradycardique étant simultanément réduit.

2. Utilisation selon la revendication 1, **caractérisée** par le fait que l'on utilise le méthylsulfate de néostigmine afin de provoquer un effet systémique agonistique-cholinergique.

3. Utilisation selon la revendication 2, **caractérisée** par le fait que ledit médicament convient au traitement de la myasthénie en réduisant les effets secondaires bradycardiques.

4. Utilisation selon la revendication 1, **caractérisée** par le fait que l'on utilise le méthylsulfate de thiémonium afin de provoquer un effet systémique anti-cholinergique.
